# FASCICULE DE BREVET EUROPEEN

(11) **EP 4 360 668 B1**
(45) Date de publication et mention de la délivrance du brevet: **12.11.2025**
(21) Numéro de dépôt: 23205974.1
(22) Date de dépôt: 26.10.2023
(51) Int. Cl.: A61L 2/18, A61M 5/00, B65B 7/28, B65B 3/00

(54) **PROCÉDÉ DE REMPLISSAGE DE SERINGUES**
VERFAHREN ZUM FÜLLEN VON SPRITZEN
METHOD FOR FILLING SYRINGES

(30) Priorité: 27.10.2022 BE 202205883
(43) Date de publication de la demande: 01.05.2024
(73) Titulaire: Cenexi - Laboratoires Thissen, 1420 Braine-l'Alleud (BE)
(72) Inventeur: Dupont, Yves, 4280 Hannut (BE)
(74) Mandataire: Gevers Patents

(56) Documents cités:
- EP-A1- 2 119 463
- EP-A1- 3 871 705
- WO-A1-2012/025549
- WO-A1-2016/166769
- WO-A1-2022/128884
- BE-A4- 1 018 901

## Description

### DOMAINE DE L'INVENTION

La présente invention concerne un procédé de remplissage de seringues en conditions aseptiques avec une composition stérile préalablement formée.

La présente invention concerne en outre une seringue remplie au moins partiellement issue du procédé selon l'invention, en particulier directement issue du procédé selon l'invention.

### CONTEXTE DE L'INVENTION

Les seringues préremplies sont fréquemment utilisées en médecine clinique pour injecter des solutions dans le corps humain, par exemple lors de l'administration de vaccins ou médicaments sous forme liquide. Il va bien entendu de soi qu'il est indispensable que les solutions contenues dans les seringues préremplies soient parfaitement stériles.

Par les termes « seringues préremplies », il est entendu, au sens de la présente invention, des seringues prêtes à l'emploi, c'est-à-dire des seringues contenant une solution prête à être injectée.

En pratique, afin d'obtenir une seringue préremplie dans laquelle une solution injectable est stérile, un corps de seringue généralement muni d'une aiguille insérée dans un capuchon de protection transite au travers d'une chaîne de remplissage afin d'y être remplie et munie d'un piston, l'ensemble des opérations étant réalisé en conditions aseptiques.

Il importe bien entendu que le corps de seringue muni d'une aiguille insérée dans un capuchon de protection soit lui-même stérile dès le départ, faute de quoi le corps de seringue lui-même serait responsable de contaminations de la solution injectable. A cette fin, comme décrit par exemple dans le document EP3269409, un dispositif particulier de conditionnement de corps de seringue existe et consiste en un boitier (ou « tub ») associé à un nest à seringues agencé pour supporter des corps de seringues. Le boitier accueille le nest à seringues de telle sorte qu'une série de corps de seringues est maintenue suspendue dans le boitier (ou « tub »), lequel boitier est fermé hermétiquement et stérilement à l'aide d'un film polymère. Les opérations relatives à un tel conditionnement de corps de seringues sont systématiquement réalisées en conditions aseptiques de telle sorte que, une fois le boitier fermé, les corps de seringues munis d'une aiguille insérée dans un capuchon de protection sont parfaitement stériles. Par mesure de précaution supplémentaire, le boitier fermé est ensaché. Cet ensachage est typiquement effectué à l'aide de machines à ensacher pour ensachage aseptique simple ou double : dans un environnement aseptique, un premier sachet est ouvert et le boitier fermé y est inséré. Par la suite, ce premier sachet est scellé hermétiquement de telle sorte qu'un premier ensachage est réalisé. Généralement, le premier sachet fermé hermétiquement et contenant le boitier fermé est placé dans un second sachet, lequel est également ensuite fermé hermétiquement de telle sorte qu'un second ensachage est réalisé.

Afin que les corps de seringues munis d'une aiguille insérée dans un capuchon de protection puissent être remplis par passage au travers d'une chaîne de remplissage, il convient en premier lieu de procéder au retrait du ou des sachet(s) pour pouvoir accéder au boitier fermé hermétiquement et stérilement à l'aide d'un film polymère. Cette opération de déballage est à ce jour effectuée manuellement avec grand soin dans un environnement stérile afin de minimiser toute contamination par contact avec le boitier fermé. Dans un second temps et dans un environnement où des conditions aseptiques plus strictes sont maintenues, le film polymère fermant le boitier est retiré, les corps de seringues étant lors accessibles en vue d'un remplissage en conditions aseptiques. Les documents suivants divulguent des procédés de conditionnement de dispositifs médicaux et de seringues : WO2012/025549, EP3871705, WO2022/128884, BE1018901, EP2119463, WO2016/166769.

Malheureusement, même en respectant toutes les mesures de précautions requises, l'opération manuelle de retrait du ou des sachet(s) pour pouvoir accéder au boiter fermé donne lieu à des contaminations non admissibles. En effet, réaliser cette opération manuellement rompt quasiment inévitablement la chaîne des conditions stériles et crée des contaminations, rendant ainsi les seringues impropres et inutilisables. Par ailleurs, cette opération de déballage manuelle est coûteuse en temps et monopolise un opérateur.

Il existe donc un réel besoin de procurer un procédé de remplissage de seringues qui permette de réduire significativement toute intervention manuelle, notamment lors du retrait du ou des sachet(s) pour pouvoir accéder au boitier fermé, qui garantisse en outre le maintien de conditions stériles lors du processus de remplissage, et qui permette de retirer le ou les sachet(s) à grande vitesse ou à débit élevé.

### RESUME DE L'INVENTION

Le procédé selon la présente invention entend répondre au moins partiellement aux problématiques mentionnées ci-dessus.

Dans un premier aspect de la présente invention, il est proposé un procédé de remplissage en conditions aseptiques de seringues avec une composition stérile préalablement formée, ledit procédé comprenant séquentiellement :
a) une étape d'amenée, dans une première zone, d'un premier ensemble comprenant un boitier et un nest à seringues supportant des corps de seringues munis d'une aiguille insérée dans un capuchon de protection, ledit nest à seringues se trouvant dans ledit boitier, lequel est fermé au niveau de sa partie supérieure à l'aide d'un film polymère, ledit premier ensemble étant ensaché dans un premier sachet lui-même ensaché dans un deuxième sachet,
b) une première étape d'ouverture dudit deuxième sachet pour atteindre ledit premier sachet,
c) une deuxième étape d'ouverture dudit premier sachet pour atteindre ledit premier ensemble comprenant ledit boitier et ledit nest à seringues,
d) une étape de transfert dudit premier ensemble vers une deuxième zone aseptique, cette étape de transfert étant réalisée à l'aide d'au moins un moyen de transfert agencé pour permettre un déplacement dudit premier ensemble depuis ladite première zone vers ladite deuxième zone aseptique,
e) une étape de retrait dudit film polymère de la partie supérieure dudit boitier pour rendre accessible ledit nest à seringues supportant des corps de seringues munis d'une aiguille insérée dans un capuchon de protection, cette étape de retrait étant réalisée à l'aide d'un moyen de retrait agencé pour permettre un retrait dudit film polymère au départ de la partie supérieure dudit boitier,
f) une première étape de préhension dudit nest à seringues pour l'extraire dudit boitier, cette première étape de préhension étant réalisée à l'aide d'un moyen de préhension agencé pour saisir ledit nest à seringues et pour l'extraire dudit boitier,
g) une étape de transfert dudit nest à seringues extrait dudit boitier vers une unité de remplissage de seringues, cette étape de transfert étant réalisée à l'aide d'au moins un moyen de transfert agencé pour permettre un déplacement dudit nest à seringues extrait dudit boitier vers une unité de remplissage de seringues,
h) une étape de remplissage desdites seringues, pour obtenir des seringues remplies au moins partiellement avec ladite composition stérile préalablement formée, cette étape de remplissage étant réalisée à l'aide d'au moins un moyen de remplissage agencé pour permettre une amenée d'une composition stérile préalablement formée dans lesdites seringues,
i) une étape de mise en place d'un bouchon interne dans chacune desdites seringues remplies au moins partiellement, cette étape étant réalisée à l'aide d'un moyen de mise en place d'un bouchon agencé pour permettre la mise en place d'un bouchon interne dans chacune desdites seringues,
j) une deuxième étape de préhension dudit nest à seringues supportant lesdites seringues remplies au moins partiellement et munies chacune d'un bouchon interne pour repositionner ledit nest à seringues dans ledit boitier et ainsi former un deuxième ensemble, cette deuxième étape de préhension étant réalisée à l'aide d'un moyen de préhension agencé pour saisir ledit nest à seringues et le déplacer de telle sorte à le repositionner dans ledit boitier,
k) une étape de transfert dudit deuxième ensemble vers une unité d'assemblage de seringues, cette étape de transfert étant réalisée à l'aide d'au moins un moyen de transfert agencé pour permettre un déplacement dudit deuxième ensemble vers une unité d'assemblage de seringues,
l) une étape d'assemblage desdites seringues remplies au moins partiellement et munies chacune d'un bouchon interne par mise en place, pour chacune des seringues, d'un dispositif de sécurité agencé pour assurer une rétraction de l'aiguille une fois ladite composition stérile injectée et d'une tige de piston se fixant au bouchon interne de telle sorte à former un piston, cette étape d'assemblage étant réalisée à l'aide d'au moins un moyen d'assemblage agencé pour permettre un assemblage desdites seringues, en particulier pour permettre la mise en place d'un dispositif de sécurité agencé pour assurer une rétraction de l'aiguille une fois ladite composition stérile injectée et pour permettre la mise en place d'une tige de piston se fixant au bouchon interne de telle sorte à former un piston,
ledit procédé étant caractérisé en ce que,
- ladite étape b) est réalisée par préhension et par maintien dudit deuxième sachet à l'aide de premiers moyens configurés pour tenir/maintenir ledit deuxième sachet dans une position telle qu'un espace est créé entre ledit deuxième sachet et ledit premier sachet, et par déplacement mécanique d'un élément d'ouverture agencé pour ouvrir ledit deuxième sachet, ledit élément d'ouverture étant mis en contact avec la surface dudit deuxième sachet et déplacé mécaniquement à la surface dudit deuxième sachet de telle sorte à pouvoir y effectuer une ouverture permettant d'atteindre ledit premier sachet, et
- ladite étape c) est réalisée par préhension et par maintien dudit premier sachet à l'aide de deuxièmes moyens configurés pour tenir/maintenir ledit premier sachet dans une position telle qu'un espace est créé entre ledit premier sachet et le ledit premier ensemble, et par déplacement mécanique d'un élément d'ouverture agencé pour ouvrir ledit premier sachet, ledit élément d'ouverture étant mis en contact avec la surface dudit premier sachet et déplacé mécaniquement à la surface dudit premier sachet de telle sorte à pouvoir y effectuer une ouverture permettant d'atteindre ledit premier ensemble.

Par les termes « ledit nest à seringues étant positionné dans ledit boitier », il est entendu, au sens de la présente invention, que le nest est situé dans le boitier, c'est-à-dire que le nest se trouve dans le boitier.

Selon l'invention, ladite première étape d'ouverture dudit deuxième sachet pour atteindre ledit premier sachet est réalisée à l'aide d'un élément d'ouverture agencé pour ouvrir ledit deuxième sachet. En particulier, ladite première étape d'ouverture dudit deuxième sachet pour atteindre ledit premier sachet est réalisée à l'aide d'un élément d'ouverture mis en contact avec la surface dudit deuxième sachet et déplacé mécaniquement à la surface dudit deuxième sachet de telle sorte à pouvoir y effectuer une ouverture permettant d'atteindre ledit premier sachet.

Selon l'invention, ladite deuxième étape d'ouverture dudit premier sachet pour atteindre ledit premier ensemble comprenant ledit boitier et ledit nest à seringues est réalisée à l'aide d'un élément d'ouverture agencé pour ouvrir ledit premier sachet. En particulier, ladite deuxième étape d'ouverture dudit premier sachet pour atteindre ledit premier ensemble est réalisée à l'aide d'un élément d'ouverture mis en contact avec la surface dudit premier sachet et déplacé mécaniquement à la surface dudit premier sachet de telle sorte à pouvoir y effectuer une ouverture permettant d'atteindre ledit premier ensemble.

Avantageusement, selon l'invention, le même élément d'ouverture est utilisé pour ouvrir en premier lieu ledit deuxième sachet et en second lieu ledit premier sachet.

Préférentiellement, selon l'invention, ledit élément d'ouverture est une lame ou tout élément permettant de réaliser une ouverture d'un sachet.

Selon l'invention, ladite étape de transfert dudit premier ensemble vers une deuxième zone aseptique est réalisée à l'aide d'au moins un moyen de transfert agencé pour permettre un déplacement dudit premier ensemble depuis ladite première zone vers ladite deuxième zone aseptique. Tout moyen de transfert adéquat comme par exemple un convoyeur ou un bras robotisé est couvert par la présente invention.

Selon l'invention, ladite étape de transfert dudit nest à seringues extrait dudit boitier vers une unité de remplissage de seringues est réalisée à l'aide d'au moins un moyen de transfert agencé pour permettre un déplacement dudit nest à seringues extrait dudit boitier vers une unité de remplissage de seringues. Tout moyen de transfert adéquat comme par exemple un convoyeur ou un bras robotisé est couvert par la présente invention.

Selon l'invention, ladite étape de transfert dudit deuxième ensemble vers une unité d'assemblage de seringues est réalisée à l'aide d'au moins un moyen de transfert agencé pour permettre un déplacement dudit deuxième ensemble vers une unité d'assemblage de seringues. Tout moyen de transfert adéquat comme par exemple un convoyeur ou un bras robotisé est couvert par la présente invention.

Selon l'invention, ladite étape de retrait dudit film polymère de la partie supérieure dudit boitier est réalisée à l'aide d'un moyen de retrait agencé pour permettre un retrait dudit film polymère au départ de la partie supérieure dudit boitier. Tout moyen de retrait comme par exemple un moyen de préhension pouvant saisir le film polymère et assurer son retrait au départ de la partie supérieure dudit boitier, par exemple une pince robotisée pouvant saisir le film polymère et assurer son retrait au départ de la partie supérieure dudit boitier, est couvert par la présente invention. Par exemple, le retrait du film polymère est réalisé à l'aide d'un moyen de retrait agencé pour saisir ledit film polymère et pour le retirer de la partie supérieure du boitier, par exemple par traction sur ledit film polymère.

Selon l'invention, ladite première étape de préhension dudit nest à seringues pour l'extraire dudit boitier est réalisée à l'aide d'un moyen de préhension agencé pour saisir ledit nest à seringues et pour l'extraire dudit boitier. Par exemple, l'extraction dudit nest à seringues au départ dudit boitier est réalisée par déplacement dudit moyen de préhension ayant saisi ledit nest à seringues.

Selon l'invention, ladite deuxième étape de préhension dudit nest à seringues pour repositionner ledit nest à seringues dans ledit boitier et ainsi former un deuxième ensemble est réalisée à l'aide d'un moyen de préhension agencé pour saisir ledit nest à seringues et le déplacer de telle sorte à le repositionner dans ledit boitier. Par exemple, le repositionnement dudit nest à seringues dans ledit boitier est réalisée par déplacement dudit moyen de préhension ayant saisi ledit nest à seringues.

Selon l'invention, ladite étape de remplissage desdites seringues est réalisée à l'aide d'au moins un moyen de remplissage agencé pour permettre une amenée d'une composition stérile préalablement formée dans lesdites seringues. Par exemple, ledit au moins un moyen de remplissage peut être un ensemble comprenant une pompe assurant un transfert d'une composition stérile préalablement formée depuis un contenant à destination des seringues, par exemple par l'intermédiaire d'au moins un tuyau.

Selon l'invention, ladite étape de mise en place d'un bouchon interne dans chacune desdites seringues remplies au moins partiellement est réalisée à l'aide d'un moyen de mise en place d'un bouchon agencé pour permettre la mise en place d'un bouchon interne dans chacune desdites seringues. Par exemple, ledit au moins un moyen de mise en place d'un bouchon est un robot assurant une insertion d'un bouchon dans chacune desdites seringues.

Selon l'invention, ladite étape d'assemblage desdites seringues est réalisée à l'aide d'au moins un moyen d'assemblage agencé pour permettre un assemblage desdites seringues, en particulier pour permettre la mise en place d'un dispositif de sécurité agencé pour assurer une rétraction de l'aiguille une fois ladite composition stérile injectée et pour permettre la mise en place d'une tige de piston se fixant au bouchon interne de telle sorte à former un piston. Par exemple, ledit au moins un moyen d'assemblage est un robot assurant la mise en place d'un dispositif de sécurité et la mise en place d'une tige de piston.

Avantageusement, le procédé tel que proposé selon la présente invention comporte toutes les étapes nécessaires pour assurer le remplissage de seringues avec une composition stérile préalablement préparée, selon ou en ligne avec les pratiques standards de l'industrie pharmaceutique.

Le procédé tel que proposé selon la présente invention permet avantageusement de réduire à un minimum le besoin d'intervention manuelle, par exemple en ayant à rectifier la position d'un boitier fermé doublement emballé vers une position correcte pour que les sachets puissent être retirés et/ou en ayant à rectifier la position d'un sachet afin de pouvoir en assurer une ouverture adéquate.

Le procédé tel que proposé selon la présente invention permet également avantageusement d'assurer un remplissage de seringues de manière aseptique selon les pratiques standards de l'industrie pharmaceutique. Plus particulièrement, les conditions aseptiques garanties lors du double emballage du boitier fermé sont maintenues lors du retrait du ou des sachet(s).

En outre, le procédé tel que proposé selon la présente invention permet avantageusement d'assurer un remplissage d'un plus grand nombre de seringues pour un laps de temps donné.

Dans un deuxième aspect de la présente invention, il est proposé une seringue remplie, au moins partiellement, issue du procédé selon l'invention, en particulier directement issue du procédé selon l'invention.

### DESCRIPTION DETAILLEE

Un aspect de l'invention concerne un procédé de remplissage en conditions aseptiques de seringues avec une composition stérile préalablement formée.

Dans une première étape a) du procédé selon l'invention, il est prévu une étape d'amenée, dans une première zone, d'un premier ensemble comprenant un boitier et un nest à seringues supportant des corps de seringues munis d'une aiguille insérée dans un capuchon de protection, ledit nest à seringues étant positionné dans ledit boitier, lequel est fermé au niveau de sa partie supérieure à l'aide d'un film polymère, ledit premier ensemble étant ensaché dans un premier sachet lui-même ensaché dans un deuxième sachet.

Dans des modes de réalisation du procédé, ladite première zone est une zone d'environnement contrôlé, de préférence une zone qui est qualifié comme ou qui correspond à un environnement cGMP (current Good Manufacturing Practices (GMP) ou bonnes pratiques de fabrication) de grade D.

Dans des modes de réalisation du procédé, ladite étape d'amenée comprend l'entrée dudit premier ensemble, ensaché dans un premier sachet lui-même ensaché dans un deuxième sachet, via un convoyeur ou via un premier système de trappe dans ladite première zone. De préférence, avant l'amenée dudit premier ensemble, l'extérieur dudit premier ensemble, plus particulièrement la couche extérieure dudit deuxième sachet est nettoyée ou décontaminée. Cela se fait typiquement par pulvérisation d'eau pour préparation injectable (eau PPI) ou d'une composition contenant un détergent sur ladite couche extérieure dudit deuxième sachet.

Au sens de la présente invention, le terme « convoyeur » désigne tout moyen ou tout dispositif permettant de déplacer et/ou de faire transiter un élément d'un premier endroit ou d'une première zone jusqu'à un deuxième endroit ou une deuxième zone.

Ledit premier ensemble comprend un boitier, également connu dans le domaine technique sous le nom de "tub", pourvu d'au moins un enfoncement, un rebord ou d'autres moyens afin d'accueillir un nest à seringues. De préférence, ledit boitier est pourvu d'un moins un rebord sur lequel un nest à seringues peut s'appuyer ou reposer.

Au sens de l'invention, le terme « nest » se rapporte à un plateau de remplissage de seringues, plus particulièrement à un plateau ou à une plaque avec une pluralité de trous/d'orifices configurés pour accueillir des seringues, plus particulièrement pour accueillir des corps de seringues, préférentiellement des corps de seringues munis d'une aiguille insérée dans un capuchon de protection.

Typiquement, ledit nest a une forme rectangulaire ou carrée, avec un motif d'un certain nombre de trous A dans une direction x et d'un certain nombre de trous B dans une direction y de la plaque.

Dans des modes de réalisation du procédé, le nest est configuré pour contenir au moins 60 seringues, de préférence au moins 100 seringues, plus préférentiellement au moins 120 seringues. Dans des modes de réalisation, le nest est configuré pour contenir au plus 300 seringues, de préférence au plus 250 seringues, plus préférentiellement au plus 200 seringues. Typiquement, le nest est configuré pour contenir 160 seringues.

Au sens de l'invention, le terme « corps de seringue » se rapporte à une partie cylindrique de la seringue, adaptée pour contenir une composition sous forme liquide, ledit corps de seringue étant pourvu, à une première extrémité, d'un embout configuré pour être connecté à une aiguille, et ledit corps de seringue étant pourvu, à une deuxième extrémité, d'une ouverture et d'au moins une ailette ou aileron, de préférence de deux ailettes ou ailerons, ladite première extrémité étant opposée à ladite deuxième extrémité.

Selon le procédé, ledit premier ensemble est ensaché dans un premier sachet.

Dans des modes de réalisation du procédé, ledit premier ensemble comprenant ledit boitier et ledit nest à seringues supportant une pluralité de corps de seringues munis d'une aiguille insérée dans un capuchon de protection, est préalablement stérilisé ou aseptisé avant d'être ensaché dans un premier sachet. Autrement dit, ledit boitier, ledit nest à seringues ainsi que les corps de seringues munis d'une aiguille insérée dans un capuchon de protection sont parfaitement stériles avant d'être ensaché dans ledit premier sachet.

Selon le procédé, ledit premier ensemble étant ensaché dans un premier sachet, est également ensaché dans un deuxième sachet.

Dans une étape ultérieure b) du procédé selon l'invention, il est prévu une première étape d'ouverture dudit deuxième sachet pour atteindre ledit premier sachet.

Dans des modes de réalisation du procédé selon l'invention, l'étape d'ouverture dudit deuxième sachet pour atteindre ledit premier sachet est effectuée manuellement. Avantageusement, cette étape d'ouverture du deuxième sachet est réalisée sans rompre les conditions aseptiques à l'intérieur dudit premier sachet.

Dans des modes alternatifs de réalisation du procédé selon l'invention, il est prévu des premiers moyens pour maintenir ledit premier ensemble, ensaché dans un premier sachet lui-même ensaché dans un deuxième sachet ou autrement dit doublement emballé, dans une position prédéterminée au moins pendant une partie de la durée de l'étape b). Lesdits premiers moyens pour maintenir ledit premier ensemble doublement emballé peuvent comporter au moins l'un des éléments choisi parmi un piston et/ou une ventouse pneumatique.

Selon l'invention, lesdits premiers moyens pour maintenir ledit premier ensemble doublement emballé sont configurés pour tenir/maintenir ledit deuxième sachet dans une position telle qu'un espace est créé entre ledit deuxième sachet et le reste dudit premier ensemble doublement emballé. Autrement dit, lesdits premier moyens pour tenir/maintenir ledit premier ensemble doublement emballé sont configurés pour écarter ledit deuxième sachet du reste dudit premier ensemble doublement emballé, en particulier pour écarter ledit deuxième sachet dudit premier sachet, afin de permettre à un premier élément d'ouverture d'agir sur ledit deuxième sachet sans compromettre l'herméticité et la stérilité dudit premier sachet.

Dans des modes de réalisation du procédé selon l'invention, un premier élément d'ouverture est prévu pour ouvrir ou découper au moins une partie du deuxième sachet enveloppant ledit premier ensemble doublement emballé. De préférence, ledit premier élément d'ouverture est configuré pour être déplacé selon un axe prédéterminé, de préférence à l'aide d'un vérin pneumatique.

De préférence, ledit premier élément d'ouverture se rapporte à un premier outil de coupe ou outil de découpe.

De préférence, ledit premier élément d'ouverture est configuré pour être connecté à un vérin pneumatique, agencé pour agir le long d'un axe prédéterminé.

Par exemple, ledit premier élément d'ouverture peut être une lame ou tout élément permettant de réaliser une ouverture d'un sachet.

Dans des modes de réalisation du procédé selon l'invention, il est prévu au moins une caméra et/ou un capteur pour évaluer et/ou contrôler la position dudit premier ensemble doublement emballé au moins avant d'opérer l'ouverture dudit deuxième sachet.

De préférence, les mesures obtenues avec ladite caméra et/ou ledit capteur sont utilisées pour guider lesdits premier moyens pour maintenir ledit premier ensemble doublement emballé dans une position adéquate, en particulier pour tenir/maintenir ledit deuxième sachet à distance dudit premier ensemble doublement emballé.

La combinaison d'une caméra et/ou d'un capteur et desdits premiers moyens pour maintenir ledit premier ensemble doublement emballé dans une position prédéterminée, en particulier pour tenir/maintenir ledit deuxième sachet à distance dudit premier ensemble doublement emballé, dans laquelle le signal de sortie de la caméra et/ou du capteur est utilisé comme signal d'entrée pour guider lesdits desdits premiers moyens pour maintenir ledit premier ensemble doublement emballé, en particulier pour tenir/maintenir ledit deuxième sachet à distance dudit premier ensemble doublement emballé, présente l'avantage que ledit premier ensemble doublement emballé peut être placé ou fixé selon une position prédéterminée avec une précision suffisante pour entamer l'ouverture dudit deuxième sachet sans compromettre l'herméticité et la stérilité dudit premier sachet.

Dans des modes de réalisation du procédé selon l'invention, ladite étape b) comprend en outre une étape ultérieure de séparation b') dudit premier ensemble emballé ou ensaché dans ledit premier sachet dudit deuxième sachet.

De préférence, ledit premier ensemble est séparé dudit deuxième sachet en retirant le premier ensemble emballé dans ledit premier sachet tout en maintenant le deuxième sachet en place.

De préférence, il est fait usage d'au moins un piston et/ou d'une ventouse pneumatique qui est en contact avec la surface dudit deuxième sachet, exerçant ainsi une sous-pression sur ladite surface et maintenant le deuxième sachet selon une position prédéterminée, en particulier dans une position maintenant au moins une partie dudit deuxième sachet à distance d'au moins une partie dudit premier sachet.

Dans des modes alternatifs de réalisation du procédé selon l'invention, il est fait usage des premiers moyens de préhension, plus particulièrement d'un premier préhenseur robotisé, attaché de préférence à un premier bras robotisé et muni de préférence d'au moins une ventouse pneumatique, pour retirer ledit premier ensemble emballé dans ledit premier sachet de sa position.

Dans des modes de réalisation du procédé selon l'invention, les actions de l'étape b) sont exécutées dans une zone qui correspond à une zone qualifiée de cGMP grade C.

Dans une étape ultérieure c) du procédé selon l'invention, il est prévu une deuxième étape d'ouverture dudit premier sachet pour atteindre ledit premier ensemble comprenant ledit boitier et ledit nest à seringues.

Dans des modes de réalisation du procédé selon l'invention, les actions de l'étape c) sont exécutées dans une zone qui correspond à une zone qualifiée de cGMP de grade B.

Dans des modes de réalisation du procédé selon l'invention, il est prévu des deuxièmes moyens pour maintenir ledit premier ensemble ensaché dans ledit premier sachet dans une position prédéterminée au moins pendant une partie de la durée de l'étape c). Lesdits deuxièmes moyens pour maintenir ledit premier ensemble ensaché dans ledit premier sachet peuvent être choisis parmi un piston et/ou une ventouse pneumatique.

Selon l'invention, lesdits deuxièmes moyens pour maintenir ledit premier ensemble ensaché dans ledit premier sachet sont configurés pour tenir/maintenir ledit premier sachet dans une position telle qu'un espace est créé entre ledit premier sachet et ledit premier ensemble. Autrement dit, lesdits deuxièmes moyens pour tenir/maintenir ledit premier ensemble ensaché dans ledit premier sachet sont configurés pour écarter ledit premier sachet du reste dudit premier ensemble, plus particulièrement pour écarter ledit premier sachet dudit boitier fermé par un film polymère, afin de permettre à un deuxième élément d'ouverture d'agir sur ledit premier sachet.

De préférence, lesdits deuxièmes moyens pour tenir/maintenir ledit premier ensemble ensaché dans ledit premier sachet utilisés à l'étape c) sont similaires ou identiques aux premiers moyens pour tenir/maintenir ledit premier ensemble doublement emballé, utilisés à l'étape b).

Dans des modes de réalisation du procédé selon l'invention, un deuxième élément d'ouverture est prévu pour ouvrir ou découper au moins une partie du premier sachet enveloppant ledit premier ensemble. De préférence, ledit deuxième élément d'ouverture est configuré pour être déplacé selon un axe prédéterminé, de préférence à l'aide d'un vérin pneumatique.

De préférence, ledit deuxième élément d'ouverture se rapporte à un deuxième outil de coupe ou outil de découpe.

De préférence, ledit deuxième élément d'ouverture est configuré pour être connecté à un vérin pneumatique, agencé pour agir le long d'un axe prédéterminé.

Par exemple, ledit deuxième élément d'ouverture peut être une lame ou tout élément permettant de réaliser une ouverture d'un sachet.

De préférence, ledit deuxième élément d'ouverture utilisé à l'étape c) est similaire ou identique au premier élément d'ouverture utilisé à l'étape b).

Dans des modes de réalisation du procédé selon l'invention, il est prévu au moins une caméra et/ou un capteur pour contrôler la position dudit premier ensemble ensaché dans ledit premier sachet au moins avant d'opérer l'ouverture dudit premier sachet.

De préférence, les mesures obtenues avec ladite caméra et/ou ledit capteur sont utilisées pour guider lesdits deuxièmes moyens pour tenir/maintenir ledit premier ensemble ensaché dans ledit premier sachet dans une position adéquate.

La combinaison d'une caméra et/ou d'un capteur et des deuxièmes moyens pour maintenir ledit premier ensemble ensaché dans ledit premier sachet, dans laquelle le signal de sortie de la caméra et/ou du capteur est utilisé comme signal d'entrée pour guider lesdits deuxièmes moyens pour maintenir ledit premier ensemble ensaché dans ledit premier sachet, présente l'avantage que le premier ensemble ensaché dans ledit premier sachet peut être placé ou fixé dans une position prédéterminée avec une précision suffisante pour entamer l'ouverture dudit premier sachet sans endommager le premier ensemble, en particulier sans endommager ni le boitier ni le film polymère fermant le boitier.

Dans des modes de réalisation du procédé selon l'invention, ladite étape c) comprend en outre une étape ultérieure de séparation c') dudit premier ensemble dudit premier sachet.

De préférence, ledit premier ensemble est séparé dudit premier sachet en retirant le premier ensemble tout en maintenant le premier sachet en place.

De préférence, il est fait usage d'au moins un piston et/ou d'au moins une ventouse pneumatique qui est en contact avec la surface dudit premier sachet, exerçant ainsi une sous-pression sur ladite surface de telle sorte à tenir/maintenir au moins une partie dudit premier sachet selon une position prédéterminée, en particulier de telle sorte à tenir/maintenir au moins une partie dudit premier sachet à distance dudit premier ensemble, en particulier à distance dudit boitier.

De préférence, il est fait usage des deuxièmes moyens de préhension, en particulier d'un deuxième préhenseur robotisé, attaché de préférence à un deuxième bras robotisé et muni de préférence d'au moins une ventouse pneumatique, pour retirer ledit premier ensemble de sa position.

De préférence, lesdits premiers moyens de préhension et lesdits deuxièmes moyens de préhension sont similaires ou identiques.

Selon le procédé de l'invention, ladite étape b) et/ou ladite étape c) est donc réalisée par préhension et par maintien dudit deuxième sachet et/ou dudit premier sachet et par déplacement mécanique d'un élément d'ouverture, agencé pour ouvrir ledit deuxième sachet et/ou ledit premier sachet.

Selon le procédé suivant l'invention, ladite première étape d'ouverture dudit deuxième sachet pour atteindre ledit premier sachet est effectuée à l'aide de premiers moyens configurés pour tenir/maintenir ledit deuxième sachet dans une position telle qu'un espace est créé entre ledit deuxième sachet et le ledit premier sachet.

Selon le procédé suivant l'invention, lesdits premiers moyens sont choisis parmi un piston et/ou une ventouse pneumatique.

Selon le procédé suivant l'invention, ladite deuxième étape d'ouverture dudit premier sachet pour atteindre ledit premier ensemble comprenant ledit boitier et ledit nest à seringues est effectuée à l'aide de deuxièmes moyens configurés pour tenir/maintenir ledit premier sachet dans une position telle qu'un espace est créé entre ledit premier sachet et le ledit premier ensemble.

Selon le procédé suivant l'invention, lesdits deuxièmes moyens sont choisis parmi un piston et/ou une ventouse pneumatique.

Dans une étape ultérieure d) du procédé selon l'invention, il est prévu une étape de transfert dudit premier ensemble vers une deuxième zone aseptique.

Dans des modes de réalisation du procédé selon l'invention, ladite deuxième zone aseptique se rapporte à une zone qualifiée de cGMP de grade A.

Dans des modes de réalisation selon l'invention, ledit transfert dudit premier ensemble vers une deuxième zone aseptique est effectué par un convoyeur.

De préférence, ledit premier ensemble passe par une trappe ou par un deuxième système de trappe, qui sépare ladite première zone de ladite deuxième zone aseptique.

Dans une étape ultérieure e) du procédé selon l'invention, il est prévu une étape de retrait dudit film polymère de la partie supérieure dudit boitier pour rendre accessible ledit nest à seringues supportant des corps de seringues munis d'une aiguille insérée dans un capuchon de protection.

Dans des modes de réalisation préférés du procédé selon l'invention, le retrait dudit film polymère de la partie supérieure dudit boitier est effectuée de manière automatisée.

Typiquement, cette étape de retrait dudit film polymère se traduira par rendre ledit nest à seringues accessible à des troisièmes moyens de préhension, en particulier à un troisième préhenseur robotisé, attaché de préférence à un troisième bras robotisé.

Dans une étape ultérieure f) du procédé selon l'invention, il est prévu une première étape de préhension dudit nest à seringues pour l'extraire dudit boitier.

Dans des modes de réalisation préférés du procédé selon l'invention, des troisièmes moyens de préhension sont utilisés pour extraire ledit nest à seringues dudit boitier.

Lesdits troisièmes moyens de préhension comportent de préférence un troisième préhenseur robotisé, attaché de préférence à un troisième bras robotisé et muni d'au moins une ventouse pneumatique.

Dans une étape ultérieure g) du procédé selon l'invention, il est prévu une étape de transfert dudit nest à seringues extrait dudit boitier vers une unité de remplissage de seringues.

De préférence, lesdits moyens de préhension comportent de préférence un préhenseur robotisé, attaché de préférence à un bras robotisé et muni d'au moins une ventouse pneumatique. Plus particulièrement, ledit préhenseur robotisé est connecté à un bras robotisé, qui est programmable pour transférer ledit nest à seringues d'un premier emplacement (typiquement dudit boitier ou « tub ») à un second emplacement, ledit second emplacement étant de préférence une unité de remplissage de seringues.

Dans une étape ultérieure h) du procédé selon l'invention, il est prévu une étape de remplissage desdites seringues, pour obtenir des seringues remplies au moins partiellement avec ladite composition stérile préalablement formée.

Selon l'invention, ladite étape de remplissage est réalisée dans une unité de remplissage de seringues, comprenant de préférence au moins une chambre de remplissage.

Dans des modes de réalisation préférés du procédé selon l'invention, l'unité de remplissage de seringues comprend des moyens agencés pour recevoir et supporter ledit nest à seringues, apporté par lesdits moyens de préhension.

De préférence, lesdits moyens agencés pour recevoir et supporter ledit nest ont la forme dudit boitier dont le nest a été enlevé.

Typiquement, lesdites ouvertures à ladite extrémité dudit corps de seringue sont tournées vers le haut lors de leur remplissage à ladite étape h).

Dans des modes de réalisation préférés du procédé selon l'invention, ladite unité de remplissage de seringues comprend des moyens de remplissage, lesdits moyens de remplissage comprenant de préférence une buse ou une aiguille dirigée vers le bas et de préférence des moyens pour faire descendre ladite buse ou ladite aiguille dirigée vers le bas de sorte qu'elle s'approche ou pénètre dans un desdits corps de seringues par ladite ouverture dudit corps de seringue, pour fournir ladite composition stérile préalablement formée dans ledit corps de seringue tout en maintenant ledit corps de seringue en position fixe.

Dans des modes de réalisation préférés du procédé selon l'invention, ladite composition stérile préalablement formée est injectée en utilisant une pompe péristaltique.

Typiquement, au moins 1 ml et au plus 70 ml de ladite composition stérile préalablement formée est injecté dans ledit corps de seringue lors de ladite étape de remplissage h).

Dans des modes de réalisation du procédé selon l'invention, ledit corps de seringue est configuré pour être rempli avec 1 ml, 3 ml, 5 ml, 10 ml, 20 ml, 30 ml ou 50 ml de ladite composition stérile préalablement formée.

Il sera clair pour l'homme du métier qu'en raison de la présence de l'aiguille insérée dans un capuchon de protection à une extrémité du corps de seringue opposée à l'autre extrémité du corps de seringue comportant l'ouverture du corps de seringue, la composition restera à l'intérieur du corps de la seringue.

Dans une étape ultérieure i) du procédé selon l'invention, il est prévu une étape de mise en place d'un bouchon interne dans chacune desdites seringues remplies au moins partiellement.

Dans des modes de réalisation préférés du procédé selon l'invention, ledit bouchon interne se rapporte à un butoir.

Dans une étape ultérieure j) du procédé selon l'invention, il est prévu une deuxième étape de préhension dudit nest à seringues supportant lesdites seringues remplies au moins partiellement et munies chacune d'un bouchon interne pour repositionner ledit nest à seringues dans ledit boitier et ainsi former un deuxième ensemble.

Dans des modes de réalisation préférés du procédé selon l'invention, des quatrièmes moyens de préhension sont utilisés pour extraire ledit nest à seringues de ladite unité de remplissage de seringues.

Lesdits quatrièmes moyens de préhension comportent de préférence un quatrième préhenseur robotisé, attaché de préférence à un quatrième bras robotisé et muni d'au moins une ventouse pneumatique.

Dans une étape ultérieure k) du procédé selon l'invention, il est prévu une étape de transfert dudit deuxième ensemble vers une unité d'assemblage de seringues.

De préférence, lesdits quatrièmes moyens de préhension comportent en outre un quatrième bras robotisé, attaché de préférence à un quatrième bras robotisé et muni d'au moins une ventouse pneumatique. Plus en particulier, ledit quatrième préhenseur robotisé est connecté à un quatrième bras robotisé, qui est programmable pour transférer ledit nest supportant lesdites seringues remplies et munies chacune d'un bouchon interne de l'unité de remplissage de seringues vers une unité d'assemblage de seringues.

Dans des modes de réalisation préférés du procédé selon l'invention, lesdits troisièmes moyens de préhension sont similaires ou identiques auxdits quatrièmes moyens de préhension. Selon ces modes de réalisation, ledit troisième préhenseur robotisé et ledit quatrième préhenseur robotisé sont similaires ou identiques. Également, ledit troisième bras robotisé et ledit quatrième bras robotisé sont similaires ou identiques.

Dans une étape ultérieure l) du procédé selon l'invention, il est prévu une étape d'assemblage desdites seringues remplies au moins partiellement et munies chacune d'un bouchon interne par mise en place, pour chacune des seringues, d'un dispositif de sécurité agencé pour assurer une rétraction de l'aiguille une fois ladite composition stérile injectée et d'une tige de piston se fixant au bouchon interne de telle sorte à former un piston.

Dans des modes de réalisation préférés du procédé selon l'invention, ladite tige de piston se fixe de telle manière à ce que ledit bouchon interne forme le butoir du piston.

Dans des modes de réalisation préférés du procédé selon l'invention, les étapes e) à l) sont exécutées dans ladite deuxième zone aseptique.

Dans des modes de réalisation préférés du procédé selon l'invention, il est prévu une étape d'inspection et/ou de contrôle avant assembler, pour chacune des seringues, ledit dispositif de sécurité et ladite tige de piston.

## Revendications

1. Procédé de remplissage en conditions aseptiques de seringues avec une composition stérile préalablement formée, ledit procédé comprenant séquentiellement :
a) une étape d'amenée, dans une première zone, d'un premier ensemble comprenant un boitier et un nest à seringues supportant des corps de seringues munis d'une aiguille insérée dans un capuchon de protection, ledit nest à seringues se trouvant dans ledit boitier, lequel est fermé au niveau de sa partie supérieure à l'aide d'un film polymère, ledit premier ensemble étant ensaché dans un premier sachet lui-même ensaché dans un deuxième sachet,
b) une première étape d'ouverture dudit deuxième sachet pour atteindre ledit premier sachet,
c) une deuxième étape d'ouverture dudit premier sachet pour atteindre ledit premier ensemble comprenant ledit boitier et ledit nest à seringues,
d) une étape de transfert dudit premier ensemble vers une deuxième zone aseptique, cette étape de transfert étant réalisée à l'aide d'au moins un moyen de transfert agencé pour permettre un déplacement dudit premier ensemble depuis ladite première zone vers ladite deuxième zone aseptique,
e) une étape de retrait dudit film polymère de la partie supérieure dudit boitier pour rendre accessible ledit nest à seringues supportant des corps de seringues munis d'une aiguille insérée dans un capuchon de protection, cette étape de retrait étant réalisée à l'aide d'un moyen de retrait agencé pour permettre un retrait dudit film polymère au départ de la partie supérieure dudit boitier,
f) une première étape de préhension dudit nest à seringues pour l'extraire dudit boitier, cette première étape de préhension étant réalisée à l'aide d'un moyen de préhension agencé pour saisir ledit nest à seringues et pour l'extraire dudit boitier,
g) une étape de transfert dudit nest à seringues extrait dudit boitier vers une unité de remplissage de seringues, cette étape de transfert étant réalisée à l'aide d'au moins un moyen de transfert agencé pour permettre un déplacement dudit nest à seringues extrait dudit boitier vers une unité de remplissage de seringues,
h) une étape de remplissage desdites seringues, pour obtenir des seringues remplies au moins partiellement avec ladite composition stérile préalablement formée, cette étape de remplissage étant réalisée à l'aide d'au moins un moyen de remplissage agencé pour permettre une amenée d'une composition stérile préalablement formée dans lesdites seringues,
i) une étape de mise en place d'un bouchon interne dans chacune desdites seringues remplies au moins partiellement, cette étape étant réalisée à l'aide d'un moyen de mise en place d'un bouchon agencé pour permettre la mise en place d'un bouchon interne dans chacune desdites seringues,
j) une deuxième étape de préhension dudit nest à seringues supportant lesdites seringues remplies au moins partiellement et munies chacune d'un bouchon interne pour repositionner ledit nest à seringues dans ledit boitier et ainsi former un deuxième ensemble, cette deuxième étape de préhension étant réalisée à l'aide d'un moyen de préhension agencé pour saisir ledit nest à seringues et le déplacer de telle sorte à le repositionner dans ledit boitier,
k) une étape de transfert dudit deuxième ensemble vers une unité d'assemblage de seringues, cette étape de transfert étant réalisée à l'aide d'au moins un moyen de transfert agencé pour permettre un déplacement dudit deuxième ensemble vers une unité d'assemblage de seringues,
l) une étape d'assemblage desdites seringues remplies au moins partiellement et munies chacune d'un bouchon interne par mise en place, pour chacune des seringues, d'un dispositif de sécurité agencé pour assurer une rétraction de l'aiguille une fois ladite composition stérile injectée et d'une tige de piston se fixant au bouchon interne de telle sorte à former un piston, cette étape d'assemblage étant réalisée à l'aide d'au moins un moyen d'assemblage agencé pour permettre un assemblage desdites seringues, en particulier pour permettre la mise en place d'un dispositif de sécurité agencé pour assurer une rétraction de l'aiguille une fois ladite composition stérile injectée et pour permettre la mise en place d'une tige de piston se fixant au bouchon interne de telle sorte à former un piston,
ledit procédé étant **caractérisé en ce que**,
- ladite étape b) est réalisée par préhension et par maintien dudit deuxième sachet à l'aide de premiers moyens configurés pour tenir/maintenir ledit deuxième sachet dans une position telle qu'un espace est créé entre ledit deuxième sachet et ledit premier sachet, et par déplacement mécanique d'un élément d'ouverture agencé pour ouvrir ledit deuxième sachet, ledit élément d'ouverture étant mis en contact avec la surface dudit deuxième sachet et déplacé mécaniquement à la surface dudit deuxième sachet de telle sorte à pouvoir y effectuer une ouverture permettant d'atteindre ledit premier sachet, et
- ladite étape c) est réalisée par préhension et par maintien dudit premier sachet à l'aide de deuxièmes moyens configurés pour tenir/maintenir ledit premier sachet dans une position telle qu'un espace est créé entre ledit premier sachet et le ledit premier ensemble, et par déplacement mécanique d'un élément d'ouverture agencé pour ouvrir ledit premier sachet, ledit élément d'ouverture étant mis en contact avec la surface dudit premier sachet et déplacé mécaniquement à la surface dudit premier sachet de telle sorte à pouvoir y effectuer une ouverture permettant d'atteindre ledit premier ensemble.

2. Procédé selon la revendication 1, dans lequel ladite étape d'amenée comprend une entrée dudit premier ensemble, ensaché dans ledit premier sachet lui-même ensaché dans ledit deuxième sachet, via un convoyeur ou via un premier système de trappe dans ladite première zone.

3. Procédé selon la revendication 1 ou 2, dans lequel ladite étape d'amenée dudit premier ensemble est précédée par une étape de nettoyage ou de décontamination de l'extérieur dudit premier ensemble, plus particulièrement par une étape de nettoyage ou de décontamination d'une couche extérieure dudit deuxième sachet.

4. Procédé selon l'une quelconque des revendications 1 à 3, dans lequel lesdits premiers moyens configurés pour tenir/maintenir ledit deuxième sachet dans une position telle qu'un espace est créé entre ledit deuxième sachet et ledit premier sachet sont choisis parmi un piston et/ou une ventouse pneumatique.

5. Procédé selon l'une quelconque des revendications 1 à 4, dans lequel lesdits deuxièmes moyens configurés pour tenir/maintenir ledit premier sachet dans une position telle qu'un espace est créé entre ledit premier sachet et le ledit premier ensemble sont choisis parmi un piston et/ou une ventouse pneumatique.

6. Procédé selon l'une quelconque des revendications 1 à 5, dans lequel ledit élément d'ouverture agencé pour ouvrir ledit deuxième sachet et/ou ledit premier sachet est une lame.

7. Procédé selon l'une quelconque des revendications 1 à 6, dans lequel ledit déplacement mécanique dudit élément d'ouverture est réalisé à l'aide d'un vérin pneumatique.

8. Procédé de remplissage selon l'une quelconque des revendications 1 à 7, dans lequel une étape d'inspection et/ou de contrôle est effectuée avant ladite étape l) d'assemblage desdites seringues.

9. Seringue remplie au moins partiellement issue du procédé selon l'une quelconque des revendications 1 à 8, en particulier directement issue du procédé selon l'une quelconque des revendications 1 à 8.

## Patentansprüche

1. Verfahren zum Befüllen von Spritzen unter aseptischen Bedingungen mit einer sterilen Zusammensetzung, die zuvor gebildet wurde, wobei das Verfahren nacheinander umfasst:
a) einen Schritt des Zuführens einer ersten Anordnung, die ein Gehäuse und einen Spritzenträger umfasst, der Spritzenkörper trägt, die mit einer Nadel versehen sind, welche in eine Schutzkappe eingeführt ist, in eine erste Zone, wobei sich der Spritzenträger in dem Gehäuse befindet, das an seinem oberen Teil mit Hilfe einer Polymerfolie verschlossen ist, wobei die erste Anordnung in einem ersten Beutel verpackt ist, der wiederum in einem zweiten Beutel verpackt ist,
b) einen ersten Schritt des Öffnens des zweiten Beutels, um an den ersten Beutel zu gelangen,
c) einen zweiten Schritt des Öffnens des ersten Beutels, um an die erste Anordnung zu gelangen, die das Gehäuse und den Spritzenträger umfasst,
d) einen Schritt des Transferierens der ersten Anordnung zu einer zweiten aseptischen Zone, wobei dieser Schritt des Transferierens mit Hilfe mindestens eines Transfermittels durchgeführt wird, das dafür eingerichtet ist, um eine Bewegung der ersten Anordnung von der ersten Zone zur zweiten aseptischen Zone zu ermöglichen,
e) einen Schritt des Entfernens der Polymerfolie vom oberen Teil des Gehäuses, um den Spritzenträger, der Spritzenkörper trägt, die mit einer Nadel versehen sind, welche in eine Schutzkappe eingeführt ist, zugänglich zu machen, wobei dieser Schritt des Entfernens mit Hilfe eines Entfernungsmittels durchgeführt wird, das dafür eingerichtet ist, um ein Entfernen der Polymerfolie vom oberen Teil des Gehäuses ausgehend zu ermöglichen,
f) einen ersten Schritt des Greifens des Spritzenträgers, um diesen aus dem Gehäuse zu entnehmen, wobei dieser erste Schritt des Greifens mit Hilfe eines Greifmittels durchgeführt wird, das dafür eingerichtet ist, um den Spritzenträger zu greifen und diesen aus dem Gehäuse zu entnehmen,
g) einen Schritt des Transferierens des aus dem Gehäuse entnommenen Spritzenträgers zu einer Spritzenbefülleinheit, wobei dieser Schritt des Transferierens mit Hilfe mindestens eines Transfermittels durchgeführt wird, das dafür eingerichtet ist, um eine Bewegung des aus dem Gehäuse entnommenen Spritzenträgers zu einer Spritzenbefülleinheit zu ermöglichen,
h) einen Schritt des Befüllens der Spritzen, um Spritzen zu erhalten, die mindestens teilweise mit der zuvor gebildeten sterilen Zusammensetzung gefüllt sind, wobei dieser Schritt des Befüllens mit Hilfe mindestens eines Befüllmittels durchgeführt wird, das dafür eingerichtet ist, um ein Zuführen einer zuvor gebildeten sterilen Zusammensetzung in die Spritzen zu ermöglichen,
i) einen Schritt des Anbringens eines Innenstopfens in jeder der mindestens teilweise gefüllten Spritzen, wobei dieser Schritt mit Hilfe eines Mittels zum Anbringen eines Stopfens durchgeführt wird, das dafür eingerichtet ist, um das Anbringen eines Innenstopfens in jeder der Spritzen zu ermöglichen,
j) einen zweiten Schritt des Greifens des Spritzenträgers, der die mindestens teilweise gefüllten und jeweils mit einem Innenstopfen versehenen Spritzen trägt, um den Spritzenträger in dem Gehäuse neu zu positionieren und so eine zweite Anordnung zu bilden, wobei dieser zweite Schritt des Greifens mit Hilfe eines Greifmittels durchgeführt wird, das dafür eingerichtet ist, um den Spritzenträger zu greifen und diesen derart zu bewegen, dass er in dem Gehäuse neu positioniert wird,
k) einen Schritt des Transferierens der zweiten Anordnung zu einer Spritzenmontageeinheit, wobei dieser Schritt des Transferierens mit Hilfe mindestens eines Transfermittels durchgeführt wird, das dafür eingerichtet ist, um eine Bewegung der zweiten Anordnung zu einer Spritzenmontageeinheit zu ermöglichen,
l) einen Schritt des Montierens der mindestens teilweise gefüllten und jeweils mit einem Innenstopfen versehenen Spritzen durch Anbringen, für jede der Spritzen, einer Sicherheitsvorrichtung, die dafür eingerichtet ist, um ein Zurückziehen der Nadel sicherzustellen, sobald die sterile Zusammensetzung injiziert wurde, und einer Kolbenstange, die derart am Innenstopfen befestigt wird, dass ein Kolben gebildet wird, wobei dieser Schritt des Montierens mit Hilfe mindestens eines Montagemittels durchgeführt wird, das dafür eingerichtet ist, um ein Montieren der Spritzen zu ermöglichen, insbesondere das Anbringen einer Sicherheitsvorrichtung zu ermöglichen, die dafür eingerichtet ist, um ein Zurückziehen der Nadel sicherzustellen, sobald die sterile Zusammensetzung injiziert wurde, und das Anbringen einer Kolbenstange zu ermöglichen, die derart am Innenstopfen befestigt wird, dass ein Kolben gebildet wird,
wobei das Verfahren **dadurch gekennzeichnet ist, dass**
- der Schritt b) durch Greifen und durch Festhalten des zweiten Beutels mit Hilfe von ersten Mitteln, die dafür konfiguriert sind, den zweiten Beutel in einer derartigen Position zu halten/festzuhalten, dass zwischen dem zweiten Beutel und dem ersten Beutel ein Raum erzeugt wird, und durch mechanisches Bewegen eines Öffnungselements durchgeführt wird, das dafür eingerichtet ist, den zweiten Beutel zu öffnen, wobei das Öffnungselement mit der Oberfläche des zweiten Beutels in Kontakt gebracht und mechanisch derart an der Oberfläche des zweiten Beutels bewegt wird, dass dort eine Öffnung vorgenommen werden kann, die es ermöglicht, an den ersten Beutel zu gelangen, und
- der Schritt c) durch Greifen und durch Festhalten des ersten Beutels mit Hilfe von zweiten Mitteln, die dafür konfiguriert sind, den ersten Beutel in einer derartigen Position zu halten/festzuhalten, dass zwischen dem ersten Beutel und der ersten Anordnung ein Raum erzeugt wird, und durch mechanisches Bewegen eines Öffnungselements durchgeführt wird, das dafür eingerichtet ist, den ersten Beutel zu öffnen, wobei das Öffnungselement mit der Oberfläche des ersten Beutels in Kontakt gebracht und mechanisch derart an der Oberfläche des ersten Beutels bewegt wird, dass dort eine Öffnung vorgenommen werden kann, die es ermöglicht, an die erste Anordnung zu gelangen.

2. Verfahren nach Anspruch 1, wobei der Schritt des Zuführens ein Eintreten der ersten Anordnung, die in dem ersten Beutel verpackt ist, der wiederum in dem zweiten Beutel verpackt ist, über einen Förderer oder über ein erstes Klappensystem in die erste Zone umfasst.

3. Verfahren nach Anspruch 1 oder 2, wobei dem Schritt des Zuführens der ersten Anordnung ein Schritt des Reinigens oder des Dekontaminierens der Außenseite der ersten Anordnung, spezieller ein Schritt des Reinigens oder des Dekontaminierens einer Außenschicht des zweiten Beutels vorausgeht.

4. Verfahren nach irgendeinem der Ansprüche 1 bis 3, wobei die ersten Mittel, die dafür konfiguriert sind, den zweiten Beutel in einer derartigen Position zu halten/festzuhalten, dass zwischen dem zweiten Beutel und dem ersten Beutel ein Raum erzeugt wird, aus einem Kolben und/oder einem pneumatischen Sauger ausgewählt sind.

5. Verfahren nach irgendeinem der Ansprüche 1 bis 4, wobei die zweiten Mittel, die dafür konfiguriert sind, den ersten Beutel in einer derartigen Position zu halten/festzuhalten, dass zwischen dem ersten Beutel und der ersten Anordnung ein Raum erzeugt wird, aus einem Kolben und/oder einem pneumatischen Sauger ausgewählt sind.

6. Verfahren nach irgendeinem der Ansprüche 1 bis 5, wobei das Öffnungselement, das dafür eingerichtet ist, um den zweiten Beutel und/oder den ersten Beutel zu öffnen, eine Klinge ist.

7. Verfahren nach irgendeinem der Ansprüche 1 bis 6, wobei die mechanische Bewegung des Öffnungselements mit Hilfe eines pneumatischen Zylinders durchgeführt wird.

8. Verfahren zum Befüllen nach irgendeinem der Ansprüche 1 bis 7, wobei vor dem Schritt I) des Montierens der Spritzen ein Inspektions- und/oder Kontrollschritt durchgeführt wird.

9. Mindestens teilweise gefüllte Spritze, die aus dem Verfahren nach irgendeinem der Ansprüche 1 bis 8 hervorgegangen ist, insbesondere direkt aus dem Verfahren nach irgendeinem der Ansprüche 1 bis 8 hervorgegangen ist.

## Claims

1. A method for filling syringes under aseptic conditions with a previously formed sterile composition, said method comprising sequentially:
a) a step of bringing, into a first zone, a first assembly comprising a housing and a syringe nest supporting syringe bodies provided with a needle inserted into a protective cap, said syringe nest being located in said housing, which is closed at its upper part by means of a polymer film, said first assembly being bagged in a first bag which is itself bagged in a second bag,
b) a first step of opening said second bag to reach said first bag,
c) a second step of opening said first bag to reach said first assembly comprising said housing and said syringe nest,
d) a step of transferring said first assembly to a second aseptic zone, this transfer step being carried out using at least one transfer means arranged to allow said first assembly to be moved from said first zone to said second aseptic zone,
e) a step of removing said polymer film from the upper part of said housing to make accessible said syringe nest supporting syringe bodies provided with a needle inserted into a protective cap, this removal step being carried out using a removal means arranged to allow removal of said polymer film from the upper part of said housing,
f) a first step of gripping said syringe nest to extract it from said housing, this first gripping step being performed using gripping means arranged to grasp said syringe nest and extract it from said housing,
g) a step of transferring said syringe nest extracted from said housing to a syringe filling unit, this transfer step being performed using at least one transfer means arranged to allow said syringe nest extracted from said housing to be moved to a syringe filling unit,
h) a step of filling said syringes to obtain syringes at least partially filled with said previously formed sterile composition, this filling step being performed using at least one filling means arranged to allow a previously formed sterile composition to be fed into said syringes,
i) a step of placing an internal stopper in each of said at least partially filled syringes, this step being carried out using a stopper placement means arranged to allow the placement of an internal stopper in each of said syringes,
j) a second step of gripping said syringe nest supporting said at least partially filled syringes, each provided with an internal stopper, to reposition said syringe nest in said housing and thus form a second assembly, this second gripping step being performed using a gripping means arranged to grasp said syringe nest and move it so as to reposition it in said housing,
k) a step of transferring said second assembly to a syringe assembly unit, this transfer step being performed using at least one transfer means arranged to allow said second assembly to be moved to a syringe assembly unit,
l) a step of assembling said syringes, which are at least partially filled and each provided with an internal stopper, by placing, for each of the syringes, a safety device arranged to ensure retraction of the needle once said sterile composition has been injected, and a piston rod attaching to the internal stopper so as to form a piston, this assembly step being carried out using at least one assembly means arranged to allow assembly of said syringes, in particular to allow the placement of a safety device designed to ensure retraction of the needle once the sterile composition has been injected and to allow the placement of a piston rod attaching to the inner stopper so as to form a piston,
said method being **characterized in that**,
- said step b) is performed by gripping and holding said second bag using first means configured to hold/maintain said second bag in such a position that a space is created between said second bag and said first bag, and by mechanically moving an opening element arranged to open said second bag, said opening element being brought into contact with the surface of said second bag and mechanically moved on the surface of said second bag such a way as to be able to make an opening therein allowing access to said first bag, and
- said step c) is performed by gripping and holding said first bag using second means configured to hold/maintain said first bag in a position such that a space is created between said first bag and said first assembly, and by mechanically moving an opening element arranged to open said first bag, said opening element being brought into contact with the surface of said first bag and mechanically moved on the surface of said first bag so as to be able to make an opening therein allowing access to said first assembly.

2. The method according to claim 1, wherein said feeding step comprises an inlet of said first assembly, bagged in said first bag itself bagged in said second bag, via a conveyor or via a first hatch system in said first zone.

3. The method according to claim 1 or 2, wherein said step of feeding said first assembly is preceded by a step of cleaning or decontaminating the outside of said first assembly, more particularly by a step of cleaning or decontaminating an outer layer of said second bag.

4. The method according to any one of claims 1 to 3, wherein said first means configured to hold/maintain said second bag in a position such that a space is created between said second bag and said first bag are selected from a piston and/or a pneumatic suction cup.

5. The method according to any one of claims 1 to 4, wherein said second means configured to hold/maintain said first bag in a position such that a space is created between said first bag and said first assembly are selected from a piston and/or a pneumatic suction cup.

6. The method according to any one of claims 1 to 5, wherein said opening element arranged to open said second bag and/or said first bag is a blade.

7. The method according to any one of claims 1 to 6, wherein said mechanical movement of said opening element is achieved by means of a pneumatic cylinder.

8. The filling method according to any one of claims 1 to 7, wherein an inspection and/or control step is performed before said step I) of assembling said syringes.

9. A syringe filled at least partially by the method according to any one of claims 1 to 8, in particular directly by the method according to any one of claims 1 to 8.
